# FASCICULE DE BREVET EUROPEEN

(11) **EP 2 222 243 B1**
(45) Date de publication et mention de la délivrance du brevet: **18.01.2017**
(21) Numéro de dépôt: 08864941.3
(22) Date de dépôt: 19.12.2008
(51) Int. Cl.: A61C 1/08

(54) **DISPOSITIF DE CENTRAGE ET DE GUIDAGE D'UN FORET DE PIECE A MAIN DENTAIRE**
VORRICHTUNG ZUR ZENTRIERUNG UND FÜHRUNG EINER BOHRKRONE EINES ZAHNÄRZTLICHEN HANDINSTRUMENTS
DEVICE FOR CENTRING AND GUIDING A DRILL BIT OF A DENTAL HANDPIECE

(30) Priorité: 20.12.2007 FR 0760175
(43) Date de publication de la demande: 01.09.2010
(73) Titulaire: ANTHOGYR, 74700 Sallanches (FR)
(72) Inventeur: LANCIEUX, Cédric, 74190 Passy (FR); RICHARD, Hervé, 73590 Notre Dame De Bellecombe (FR)
(74) Mandataire: Cabinet Poncet
(86) Numéro de dépôt international: PCT/IB2008/055471
(87) Numéro de publication internationale: WO 2009/081375

(56) Documents cités:
- EP-A- 1 733 695
- EP-A- 1 759 658
- WO-A-00/74585
- WO-A-03/071972
- WO-A-2004/098435
- FR-A- 2 882 250

## Description

### DOMAINE TECHNIQUE DE L'INVENTION

La présente invention concerne l'implantologie dentaire, et plus particulièrement l'implantologie dentaire assistée à l'aide d'un guide chirurgical installé en bouche du patient.

Afin de remplacer des dents endommagées dans la bouche d'un patient, on a depuis de nombreuses années recours à des implants fixés dans l'os maxillaire du patient. Après forage d'un trou dans l'os maxillaire du patient à l'aide d'une pièce à main dentaire, un implant est vissé dans le trou selon sa partie proximale pour recevoir ensuite une prothèse dentaire sur sa partie distale.

Pour aider le praticien dans la réalisation des trous dans l'os maxillaire du patient, on utilise depuis plusieurs années déjà des guides chirurgicaux que le praticien peut installer en bouche sur une mâchoire du patient. Un tel guide chirurgical est une structure en arc qui suit la courbure de la mâchoire et qui est munie d'un ou plusieurs canons tubulaires de perçage qui s'étendent jusqu'au voisinage de la zone à forer de l'os maxillaire. La réalisation de ces guides chirurgicaux est effectuée à partir de radiographies tridimensionnelles de la mâchoire du patient, ce qui permet de disposer les canons tubulaires de perçage de façon très précise.

Ces canons tubulaires de perçage comportent un alésage intérieur de diamètre constant et standardisé. A ce jour, les diamètres les plus courants sont de 4,2 mm et de 5,2 mm par exemple.

Il se pose le problème que, lors de la réalisation d'un forage dans l'os maxillaire du patient pour la pose d'un implant, le praticien utilise successivement une pluralité de forets de diamètres différents et qu'il entraîne en rotation par la pièce à main dentaire pour effectuer un trou d'abord de faible diamètre qu'il agrandit ensuite pour arriver jusqu'au diamètre nominal de l'implant à installer et qui correspond généralement au diamètre de l'alésage intérieur du canon tubulaire de perçage, à savoir par exemple 4,2 mm ou 5,2 mm pour les plus courants.

Le forage en plusieurs passes au moyen de forets de diamètres de plus en plus grands permet de limiter l'échauffement de l'os maxillaire pour éviter l'apparition de nécroses.

Afin de forer de façon précise un trou dans l'os maxillaire du patient, il est nécessaire d'assurer une bonne précision géométrique en garantissant notamment une parfaite coaxialité des forets successifs vis-à-vis du canon tubulaire de perçage.

Pour permettre l'utilisation d'une pluralité de forets de diamètres extérieurs différents et une bonne précision géométrique, on a proposé l'utilisation d'une pluralité de manchons de guidage tubulaires, aptes à s'engager chacun à faible jeu dans le canon tubulaire de perçage, et ayant chacun un alésage interne de diamètre constant sensiblement égal au diamètre extérieur du foret utilisé. Ainsi, à chaque foret de diamètre particulier correspond un manchon de guidage tubulaire apte à coopérer avec le canon tubulaire de perçage du guide chirurgical.

Une telle solution a pour inconvénient de multiplier le nombre de pièces nécessaires, et oblige les fabricants de matériel dentaire à utiliser des codes de couleurs pour aider le praticien à différencier les différentes tailles de manchon de guidage tubulaire. Mais il y a toujours un risque qu'un praticien utilise un manchon de guidage tubulaire à alésage interne de diamètre supérieur au diamètre extérieur du foret, ce qui peut conduire au forage d'un trou non coaxial au canon tubulaire de perçage du guide chirurgical.

A ce problème de maintien coaxial de forets de diamètres extérieurs différents vient s'ajouter le problème de réaliser des perçages de hauteur choisie et progressive au cours de la réalisation du trou d'implant, toujours dans le but d'éviter les nécroses.

Pour ce faire, on a alors décliné chaque manchon de guidage tubulaire à alésage interne de diamètre particulier adapté à un foret en une pluralité de manchons tubulaires de guidage de même diamètre intérieur mais de hauteurs différentes, aptes à jouer le rôle de butées positives en fin de pénétration du foret dans l'os maxillaire du patient lorsque celui-ci est à une profondeur voulue.

Dans le cadre d'une opération, le praticien a ainsi recours à de trop nombreuses pièces, ce qui augmente dramatiquement le risque d'erreurs et le risque de contamination.

Le document EP 1 759 658 propose d'utiliser plusieurs forets à épaulement, les forets ayant des longueurs différentes. L'épaulement est fixe sur les forets. Si un praticien cherche à forer l'os maxillaire en n passes de profondeurs progressives et en p passes de diamètres progressifs, le nombre théorique de forets nécessaires est de n x p, ce qui signifie de trop nombreux forets, un coût trop élevé et des risques d'erreurs et de contamination trop importants.

Le document EP 1 733 695 cherche à garantir un espace intermédiaire entre deux implants dans la mâchoire d'un patient. Il n'est pas décrit de guide chirurgical lors du forage : il n'est donc pas question, dans ce document, de garantir une parfaite coaxialité entre un foret de perçage et un canon tubulaire de perçage d'un guide chirurgical.

Le document FR 2 882 250 décrit un dispositif de centrage et de guidage d'un foret dans un canon tubulaire de perçage par l'intermédiaire d'un manchon de guidage coulissant librement sur le foret.

### EXPOSE DE L'INVENTION

Un premier problème proposé par l'invention est de concevoir un dispositif de centrage et de guidage longitudinal d'au moins un foret de pièce à main dentaire lors du forage d'un trou dans la mâchoire d'un patient en coopération avec un guide chirurgical muni d'un canon tubulaire de perçage, qui soit simple et fiable de conception, sans risque d'erreur pour le praticien, et qui assure un centrage et un guidage fiables du foret.

Simultanément, la présente invention vise à concevoir un dispositif de centrage et de guidage longitudinal utilisable avec une pluralité de forets de différents diamètres extérieurs.

L'invention cherche en outre à limiter au minimum le nombre de pièces à utiliser par le praticien pour la réalisation de forages pour l'installation d'implants dentaires en bouche d'un patient, tout en conservant une grande fiabilité de positionnement géométrique du forage et une grande fiabilité de sa profondeur.

Pour atteindre ces objets ainsi que d'autres, l'invention propose un dispositif de centrage et de guidage d'au moins un foret de pièce à main dentaire lors du forage d'un trou pour un implant dans la mâchoire d'un patient en coopération avec un guide chirurgical muni d'un canon tubulaire de perçage à extrémité supérieure d'ouverture ; selon l'invention :
- on prévoit un manchon de guidage tubulaire, apte à s'engager à faible jeu dans le canon tubulaire de perçage,
- des moyens de support maintiennent en permanence le manchon de guidage tubulaire en position coaxiale et à coulissement par rapport audit au moins un foret,
- les moyens de support sont aptes à provoquer l'engagement du manchon de guidage dans le canon tubulaire de perçage au plus tard dès que ledit au moins un foret commence à forer la mâchoire du patient.

Un tel dispositif répond efficacement aux problèmes énoncés ci-dessus.

Selon un premier mode de réalisation de l'invention, on peut prévoir que :
- ledit au moins un foret comporte une extrémité proximale et une extrémité distale libre, avec un tronçon proximal de connexion à la pièce à main dentaire et un tronçon distal de coupe,
- les moyens de support comprennent, sur ledit au moins un foret, un tronçon intermédiaire cylindrique lisse de diamètre extérieur sensiblement égal ou peu inférieur au diamètre intérieur du manchon de guidage et sur lequel est engagé à coulissement le manchon de guidage tubulaire.

De préférence, le dispositif de centrage et de guidage peut comprendre une pluralité de forets à tronçon distal de coupe de différents diamètres et comprenant tous un tronçon intermédiaire cylindrique lisse de même diamètre extérieur sensiblement égal ou peu inférieur au diamètre intérieur du manchon de guidage.

Un tel dispositif de centrage et de guidage ne nécessite l'utilisation que d'un seul et même manchon tubulaire de guidage, adapté au diamètre intérieur du canon de perçage, et utilisable avec un foret ou avec une pluralité de forets à tronçon distal de coupe de diamètres extérieurs différents. Le nombre d'éléments nécessaires pour le praticien pour assurer le guidage et une bonne précision de forage est ainsi extrêmement réduit.

Un tel dispositif de centrage et de guidage est très simple de conception, est très simple à manipuler par le praticien, limite très fortement le risque d'erreur possible dans l'orientation géométrique du forage, et a un très faible encombrement pour ne pas gêner le praticien.

De préférence, le manchon de guidage peut comporter des moyens de pénétration qui facilitent son engagement dans le canon tubulaire de perçage, de préférence une extrémité distale biseautée.

Avantageusement, le manchon de guidage peut comporter des moyens de frottement sur le tronçon intermédiaire cylindrique lisse lors de son coulissement sur celui-ci, de préférence un joint torique de diamètre intérieur sensiblement égal au diamètre extérieur du tronçon intermédiaire cylindrique lisse et logé dans une gorge annulaire intérieure du manchon de guidage.

De tels moyens de pénétration et/ou de tels moyens de frottement permettent de faciliter la pénétration du manchon de guidage tubulaire dans le canon tubulaire de perçage sans que celui-ci n'ait tendance trop facilement à remonter le long du foret en cas de défaut de coaxialité entre le manchon de guidage et le canon tubulaire de perçage au moment où le praticien commence à les faire coopérer par emboîtement.

L'usage de simples moyens de frottement, tels qu'un joint torique par exemple, permet au praticien de commencer à faire coulisser le manchon de guidage sur le tronçon intermédiaire cylindrique lisse du foret en appliquant une première force donnée, et de poursuivre ensuite ce coulissement relatif entre le manchon de guidage et le foret par l'application d'une deuxième force sensiblement identique à la première force. Il n'y a donc pas d'à-coup du fait d'une variation brusque de la force induite par les moyens de frottement en réaction à l'effort du praticien, ce qui nuirait à la qualité et à la précision du forage.

De préférence, on peut prévoir que :
- en position sur la mâchoire du patient, le guide chirurgical maintient l'extrémité supérieure d'ouverture du canon tubulaire de perçage à une première hauteur déterminée de l'extrémité distale de l'implant en fin de vissage dans la mâchoire du patient, la première hauteur étant de préférence de 9 mm environ,
- le tronçon distal de coupe a une longueur axiale inférieure à la première hauteur, comprise de préférence entre environ 4 mm et 7 mm environ,
- le tronçon distal de coupe a un diamètre extérieur supérieur au diamètre extérieur du tronçon intermédiaire lisse.

Dès lors que le tronçon distal de coupe comporte un diamètre extérieur supérieur au diamètre extérieur du tronçon intermédiaire lisse, le praticien peut aisément positionner le manchon de guidage en butée contre le tronçon distal de coupe du foret avant le forage. La longueur axiale du tronçon distal de coupe par rapport à la première hauteur déterminée permet, telle que choisie, d'assurer un engagement du manchon de guidage tubulaire dans le canon tubulaire de perçage d'au minimum deux à cinq millimètres avant que le foret ne vienne toucher la mâchoire du patient.

Avantageusement, on peut prévoir que :
- en position sur la mâchoire du patient, le guide chirurgical maintient l'extrémité supérieure d'ouverture du canon tubulaire de perçage à une première hauteur déterminée de l'extrémité distale de l'implant en fin de vissage dans la mâchoire du patient, la première hauteur étant de préférence de 9 mm environ,
- le manchon de guidage comporte une extrémité proximale de diamètre extérieur supérieur au diamètre intérieur du canon tubulaire de perçage, avec un épaulement à face de butée orientée en direction de son extrémité distale et destiné à venir en appui contre l'extrémité supérieure d'ouverture du canon tubulaire de perçage en fin de pénétration du manchon de guidage dans le canon tubulaire de perçage,
- la face de butée de l'épaulement du manchon de guidage est distante de l'extrémité distale du manchon de guidage d'une longueur inférieure à la première hauteur, avantageusement comprise entre environ 4 mm et environ 8 mm, et de préférence de 8 mm environ.

Une fois l'épaulement du manchon de guidage en butée contre l'extrémité supérieure d'ouverture du canon tubulaire de perçage, on crée, par l'intermédiaire des moyens de frottement sur le tronçon intermédiaire cylindrique lisse du foret, une force de réaction venant s'opposer à l'effort qu'applique le praticien pour faire pénétrer le foret dans l'os maxillaire du patient. Une telle force de réaction permet au praticien de mieux appréhender le stade de forage auquel il se trouve et de limiter la vitesse de pénétration dans l'os maxillaire pour éviter de l'endommager.

Enfin, la face de butée de l'épaulement du manchon de guidage étant distante de l'extrémité distale du manchon de guidage d'une longueur inférieure à la première hauteur, il est impossible que l'extrémité distale du manchon de guidage vienne au contact de la mâchoire du patient, ce qui permet de garantir une bonne hygiène et d'éviter que le manchon de guidage ne vienne endommager la zone d'opération.

Avantageusement, on peut prévoir en outre des moyens réglables de limitation de la profondeur de pénétration du foret dans la mâchoire du patient, de préférence des moyens de butée rapportés et fixés sur la pièce à main dentaire et destinés à venir en appui sur l'extrémité proximale du manchon de guidage ou sur l'extrémité supérieure d'ouverture du canon tubulaire de perçage en fin de pénétration du foret dans la mâchoire du patient.

De tels moyens réglables de limitation de la hauteur de pénétration du foret peuvent être de type connu, et on peut pour ceci avoir recours aux dispositifs de limitation et de butée généralement fournis avec les pièces à main dentaires. Le praticien peut ainsi utiliser un système qui lui est familier et bien adapté à sa pièce à main dentaire. On pourra par exemple utiliser un dispositif du type de celui décrit dans la demande de brevet français N° 06 54131 ou dans la demande de brevet français FR 2 880 534 A1.

Avantageusement, on peut prévoir des moyens de rappel élastique sollicitant en permanence le manchon de guidage en coulissement vers l'extrémité distale libre du foret.

Selon un second mode de réalisation de l'invention, on peut prévoir que :
- le manchon de guidage est déplaçable sur les moyens de support entre une position basse et une position haute,
- les moyens de support sont rapportés et fixés sur la pièce à main dentaire, et comportent des moyens de réglage permettant de régler la position axiale du manchon de guidage par rapport au foret lorsque le manchon de guidage est en position basse,
- en position haute, des premiers moyens de butée empêchent le manchon de guidage de se déplacer plus encore au-delà de la position haute en direction de l'extrémité proximale du foret.

Une telle solution permet d'utiliser un manchon de guidage unique, solidaire de la pièce à main dentaire et utilisable avec plusieurs forets standards de diamètres extérieurs différents et inférieurs au diamètre intérieur du manchon de guidage.

Le réglage de la position basse permet de situer convenablement l'extrémité distale du manchon de guidage par rapport à l'extrémité distale du foret pour assurer un engagement de celle-ci dans le canon de perçage dès le début du forage.

Le déplacement entre la position basse et la position haute permet de déplacer relativement l'extrémité distale du foret par rapport au manchon de guidage pour effectuer le forage selon la profondeur désirée.

De préférence, on peut prévoir des moyens élastiques, portés par les moyens de support, qui rappellent en permanence le manchon de guidage en position basse en direction de l'extrémité distale libre du foret.

De tels moyens élastiques permettent d'assurer un positionnement sûr du manchon de guidage dès le début du forage, sans aucune intervention du praticien. On limite ainsi efficacement tout risque que le foret vienne au contact de l'os maxillaire du patient sans être guidé convenablement.

De tels moyens élastiques permettent également de maintenir, lors du retrait axial du foret hors de la mâchoire du patient, le manchon de guidage engagé dans le canon tubulaire de perçage pendant la majeure partie du retrait du foret. On assure ainsi une bonne coaxialité du foret avec le canon tubulaire du perçage, tant lors du forage que lors de la majeure partie du retrait du foret hors de la mâchoire du patient. On limite ainsi le risque que, lors de son retrait, le foret vienne détériorer les parois du trou réalisé.

Avantageusement, on peut prévoir que :
- le guide chirurgical maintient l'extrémité supérieure d'ouverture du canon tubulaire de perçage à une première hauteur déterminée de l'extrémité distale de l'implant en fin de vissage dans la mâchoire du patient, la première hauteur étant de préférence de 9 mm environ,
- le manchon de guidage comporte une longueur axiale inférieure à la première hauteur, avantageusement comprise entre environ 4 mm et 8 mm environ,
- le manchon de guidage est solidaire, à son extrémité proximale, de seconds moyens de butée destinés à venir en appui contre l'extrémité supérieure d'ouverture du canon tubulaire de perçage en fin de pénétration du manchon de guidage dans le canon tubulaire de perçage.

On évite ainsi tout risque que l'extrémité distale du manchon de guidage vienne au contact de la mâchoire du patient, ce qui garantit une bonne hygiène et évite tout risque de lésion de la zone d'opération.

De préférence, on peut prévoir que :
- les seconds moyens de butée comprennent une plaque de butée s'étendant radialement à l'écart de l'axe longitudinal du manchon de guidage tubulaire,
- les premiers moyens de butée comprennent une plaque de support, parallèle à la plaque de butée et située dans un plan décalé longitudinalement par rapport à la plaque de butée,
- les moyens de support comprennent deux colonnes de guidage parallèles, assurant une liaison glissière entre la plaque de support et la plaque de butée,
- les moyens élastiques comprennent deux ressorts hélicoïdaux, intercalés entre les plaques de butée et de support, et rappelant les plaques de support et de butée l'une à l'écart de l'autre,
- les moyens de support comprennent une tige de support longitudinale, solidaire de la plaque de support et destinée à être fixée sur la pièce à main dentaire.

Avantageusement, on peut prévoir que :
- la tige de support longitudinale est une tige à crémaillère,
- la pièce à main dentaire comporte un manchon de réception longitudinal de la tige de support longitudinale et des moyens de blocage coopérant avec la crémaillère de la tige de support longitudinale,
- le manchon de réception longitudinal comporte un alésage interne à section transversale complémentaire en forme de la section transversale de la tige de support longitudinale,
- les sections transversales de la tige de support longitudinale et de l'alésage interne du manchon de réception longitudinal empêchent tout mouvement de rotation relatif entre ceux-ci.

Dans l'un et l'autre des premier et second modes de réalisation de l'invention, on peut prévoir que les moyens de support soient aptes à provoquer l'engagement du manchon de guidage dans le canon tubulaire de perçage selon une longueur sensiblement égale au diamètre intérieur du canon tubulaire de perçage, de préférence supérieure au diamètre intérieur du canon tubulaire de perçage, au plus tard avant que ledit au moins un foret commence à forer la mâchoire du patient.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue de côté d'un foret utilisé dans un premier mode de réalisation de l'invention ;
- la figure 2 est une vue en coupe partielle d'un manchon de guidage utilisé dans le premier mode de réalisation de l'invention ;
- la figure 3 est une vue de côté illustrant l'utilisation d'un dispositif de centrage et de guidage selon le premier mode de réalisation de l'invention ;
- la figure 4 est une vue en perspective d'une variante du premier mode de réalisation ;
- la figure 5 est une vue en perspective d'un dispositif de centrage et de guidage selon un second mode de réalisation ne faisant pas partie de l'invention, avec le manchon de guidage en position basse ;
- la figure 6 est une vue en perspective du dispositif de la figure 5, avec le manchon de guidage en position haute ;
- la figure 7 est une vue en coupe longitudinale du manchon de guidage du second mode de réalisation ne faisant pas partie de l'invention ;
- la figure 8 est une vue en perspective illustrant l'utilisation du dispositif des figures 5 et 6 ;
- la figure 9 est une vue de côté illustrant l'utilisation du dispositif des figures 5 et 6 ; et
- la figure 10 est une vue schématique en coupe partielle d'un guide chirurgical installé sur la mâchoire d'un patient.

### DESCRIPTION DES MODES DE REALISATION PREFERES

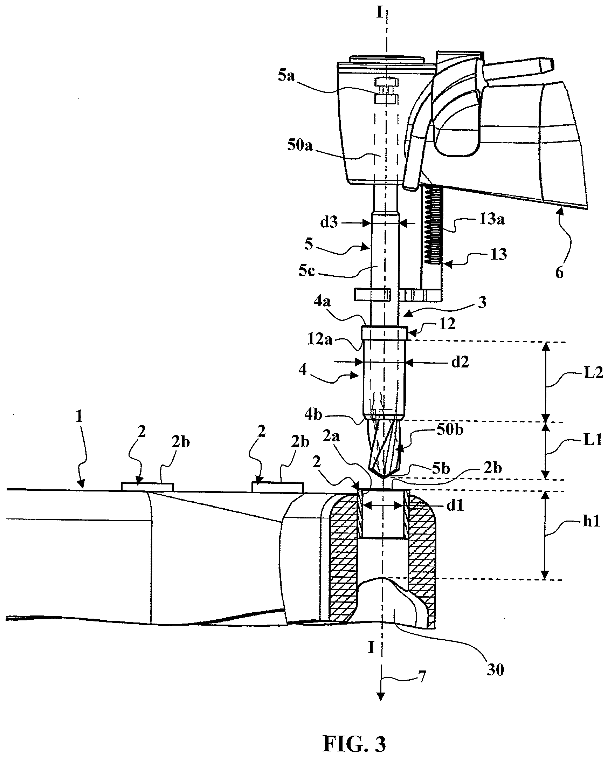

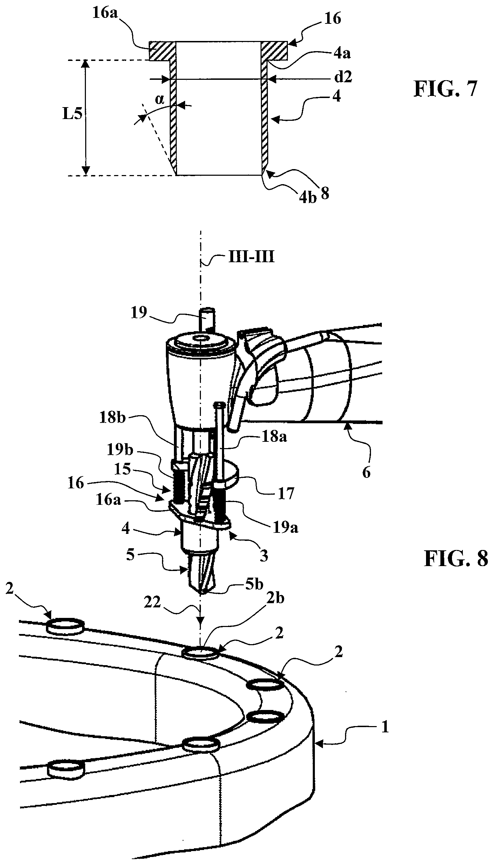

Les figures 3 et 8 montrent un guide chirurgical 1 muni d'une pluralité de canons tubulaires de perçage 2 à extrémité supérieure d'ouverture 2b. Le guide chirurgical 1 permet à un praticien d'effectuer le forage d'un trou dans la mâchoire 30 d'un patient à l'aide d'un foret 5 porté par une pièce à main dentaire 6. Les canons tubulaires de perçage 2 permettent de situer précisément l'endroit où doivent être réalisés les trous pour l'installation des implants en bouche du patient. Les canons tubulaires de perçage 2 comportent un alésage interne 2a de diamètre d1. Le diamètre d1 a une taille standardisée, et les valeurs les plus courantes sont de 4,2 mm et 5,2 mm.

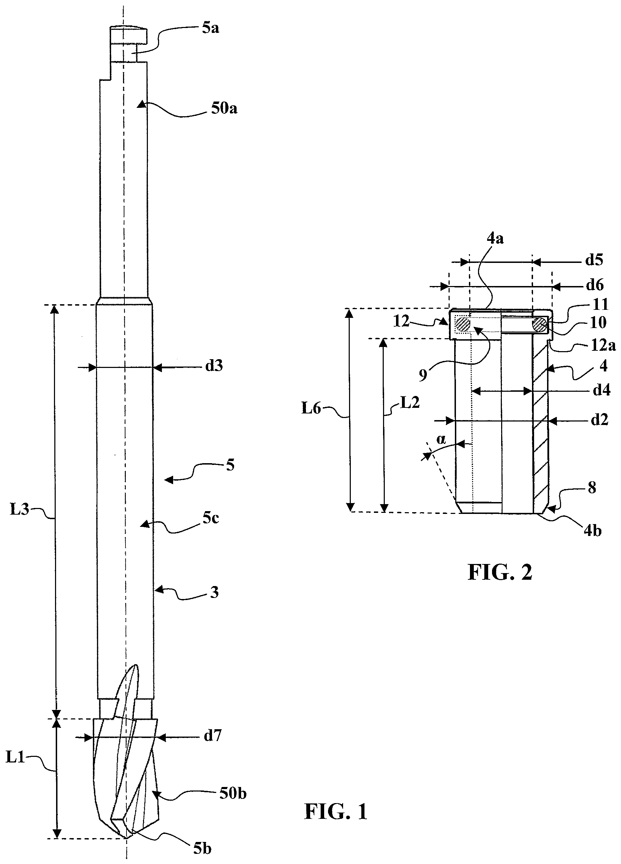

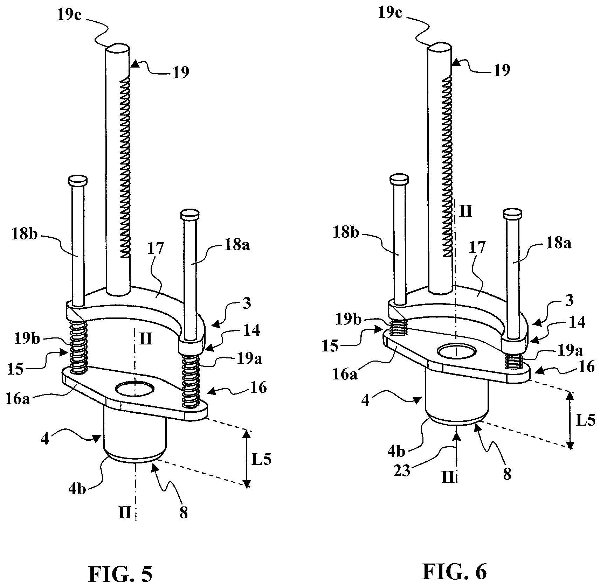

Pour réaliser avec précision le trou dans la mâchoire du patient en assurant une bonne coaxialité du trou avec le canon tubulaire de perçage 2, l'invention prévoit un dispositif de centrage et de guidage de foret. Deux modes de réalisation différents d'un tel dispositif sont respectivement illustrés par les figures 1 à 4 d'une part et 5 à 9 d'autre part. Le deuxième mode de réalisation, illustré sur les figures 5 à 9, ne fait pas partie de l'invention.

Dans ces deux modes de réalisation de l'invention, le dispositif de centrage et de guidage comporte des moyens de support 3 qui maintiennent en permanence un manchon de guidage 4 tubulaire, en position coaxiale et à coulissement par rapport à au moins un foret 5.

Le manchon de guidage 4 tubulaire est apte à s'engager à faible jeu dans le canon tubulaire de perçage 2 et présente pour ce faire un diamètre extérieur d2 très légèrement inférieur au diamètre d1. A titre d'exemple (non limitatif), on a utilisé avec de bons résultats des manchons de guidage 4 de diamètre extérieur d2 de 4,17 mm et 5,17 mm pour des canons tubulaires de perçage 2 à diamètre d1 de respectivement 4,2 mm et 5,2 mm.

Une fois le manchon de guidage 4 tubulaire engagé dans le canon tubulaire de perçage 2, celui-ci assure un centrage et un guidage longitudinal du foret 5 de façon parfaite par l'intermédiaire des moyens de support 3. Lors de l'utilisation des dispositifs de centrage et de guidage selon l'invention, les moyens de support 3 permettent l'engagement du manchon de guidage 4 dans le canon tubulaire de perçage 2 au plus tard dès que le foret 5 commence à forer la mâchoire 30 du patient.

Dans le cas du premier mode de réalisation de l'invention (figures 1 à 4), le foret 5 comporte une extrémité proximale 5a et une extrémité distale libre 5b, avec un tronçon proximal de connexion 50a à la pièce à main dentaire 6 et un tronçon distal de coupe 50b.

Les moyens de support 3 comprennent, sur le foret 5, un tronçon intermédiaire 5c cylindrique lisse de diamètre extérieur d3 sensiblement égal ou peu inférieur au diamètre intérieur d4 du manchon de guidage 4 et sur lequel peut être engagé à coulissement le manchon de guidage 4 tubulaire.

Lors de l'utilisation du premier mode de réalisation de l'invention (illustré sur la figure 3), le manchon de guidage 4 est disposé à coulissement sur le tronçon intermédiaire 5c du foret 5 et permet de guider celui-ci lors de sa pénétration dans la mâchoire 30 du patient selon un mouvement illustré par la flèche 7 selon la direction longitudinale I-I.

Le manchon de guidage 4 est utilisable avec une pluralité de forets 5 à tronçons distaux de coupe 50b de différents diamètres extérieurs d7 (figure 1), du moment que ceux-ci comprennent tous un tronçon intermédiaire 5c cylindrique lisse de même diamètre extérieur d3 sensiblement égal ou peu inférieur au diamètre intérieur d4 du manchon de guidage 4.

On permet ainsi au praticien, avec un seul et même manchon de guidage 4, d'utiliser successivement plusieurs forets 5 pour réaliser un trou en plusieurs passes à des diamètres et profondeurs progressifs, évitant ainsi de nécroser l'os maxillaire du patient au voisinage du trou.

On voit plus particulièrement sur la figure 2 que le manchon de guidage 4 comporte des moyens de pénétration 8 pour faciliter son engagement dans le canon tubulaire de perçage 2. En l'espèce, le manchon de guidage 4 est pourvu d'une extrémité distale 4b biseautée avec un chanfrein d'angle α d'environ 30° par exemple.

Pour assurer un positionnement fiable du manchon de guidage 4 sur le tronçon intermédiaire 5c du foret 5 en début de forage, le manchon de guidage 4 comporte des moyens de frottement 9 destinés à frotter sur le tronçon intermédiaire 5c cylindrique lisse. En l'espèce, le manchon de guidage 4 comporte un joint torique 10 de diamètre intérieur d5 peu inférieur ou sensiblement égal au diamètre extérieur d3 du tronçon intermédiaire 5c. Le joint torique 10 est logé et retenu captif dans une gorge annulaire intérieure 11 du manchon de guidage 4.

Le manchon de guidage 4 comporte également une extrémité proximale 4a de diamètre extérieur d6 supérieur au diamètre intérieur d1 du canon tubulaire de perçage 2, avec un épaulement 12 à face de butée 12a orientée en direction de l'extrémité distale 4b. L'épaulement 12 est destiné à venir en appui contre l'extrémité supérieure d'ouverture 2b du canon tubulaire de perçage 2 en fin de pénétration du manchon de guidage 4 dans le canon tubulaire de perçage 2.

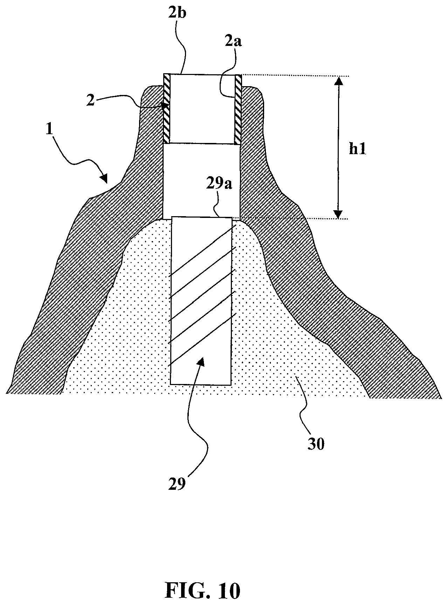

On voit plus particulièrement sur la figure 10 que le guide chirurgical 1 maintient l'extrémité supérieure d'ouverture 2b du canon tubulaire de perçage 2 à une première hauteur h1 déterminée de l'extrémité distale 29a de l'implant 29 en fin de vissage dans la mâchoire 30 du patient. De façon standard et bien connue de l'homme du métier, la première hauteur h1 est de 9 mm environ.

Pour assurer un bon guidage du foret 5 ainsi que de bonnes conditions d'hygiène, le tronçon distal de coupe 50b a une longueur axiale L1 (figures 1 et 3) inférieure à la première hauteur h1 (figure 3), et comprise de préférence entre environ 4 mm et environ 7 mm. La face de butée 12a de l'épaulement 12 du manchon de guidage 4 est quant à elle distante de l'extrémité distale 4b du manchon 4 d'une longueur L2 (figures 2 et 3) inférieure également à la première hauteur h1 (figure 3) et avantageusement comprise entre environ 4 mm et environ 8 mm. De préférence, la longueur L2 est égale à 8 mm environ lorsque la première hauteur h1 est de 9 mm.

Avantageusement, le tronçon distal de coupe 50b du foret 5 a un diamètre extérieur d7 supérieur au diamètre extérieur d3 du tronçon intermédiaire lisse 5c et au diamètre du tronçon proximal de connexion 50a qui lui-même a un diamètre inférieur ou égal au diamètre d3 du tronçon intermédiaire lisse 5c, comme illustré sur les figures. En pratique, cela permet au praticien d'insérer le manchon de guidage 4 sur le foret 5 jusqu'à faire porter l'extrémité distale 4b en butée contre le tronçon distal de coupe 50b du foret 5 avant d'engager le foret 5 dans un canon tubulaire de perçage 2, comme illustré plus particulièrement sur la figure 3.

Par une telle disposition du manchon de guidage 4 et par un tel choix des longueurs L1 et L2, on permet le guidage fiable du foret 5 avant même que celui-ci ne commence à forer la mâchoire du patient, le tronçon de guidage étant engagé en partie dans le canon tubulaire de perçage 2 avant même que le foret 5 ne commence à forer la mâchoire du patient.

La longueur L2 étant inférieure à la première hauteur h1, le manchon de guidage 4 ne peut pas venir au contact de la zone opérée de la mâchoire du patient, ce qui permet en outre de garantir une bonne hygiène.

L'installation du manchon de guidage 4 sur le tronçon intermédiaire lisse 5c du foret 5 se fait en l'insérant sur le foret 5 depuis son extrémité proximale 5a. On ne vient donc pas frotter le manchon de guidage 4 sur le tronçon distal de coupe 50b, ce qui risquerait d'endommager le manchon de guidage 4 et/ou les arêtes de coupe du foret 5. On évite également, le cas échéant, d'endommager les moyens de frottement 9, tel qu'un joint torique 10 avec les arêtes de coupe du foret 5, ce qui risquerait de produire des particules de joint qui pourraient venir polluer le forage dans la mâchoire et provoquer une infection ou autre réaction intempestive de l'organisme du patient.

Lors de l'utilisation du manchon de guidage 4, les moyens de frottement 9 ne sont jamais en contact avec les arêtes de coupe du foret 5 afin de ne pas les endommager.

Afin de contrôler la profondeur de forage du foret 5, on prévoit, comme illustré sur la figure 3, des moyens réglables de limitation de la profondeur de pénétration du foret 5 dans la mâchoire 30 du patient. En l'espèce, on utilise des moyens de butée 13 rapportés et fixés sur la pièce à main dentaire 6 et destinés à venir en appui sur l'extrémité proximale 4a du manchon de guidage 4 ou sur l'extrémité supérieure d'ouverture 2b du canon tubulaire de perçage 2 en fin de pénétration du foret 5 dans la mâchoire 30 du patient.

Pour mettre en place les implants des types les plus courants, il est nécessaire de réaliser un forage de profondeur comprise entre environ 8 mm et 15 mm environ. Le tronçon intermédiaire 5c s'étend ainsi, depuis le tronçon distal de coupe 50b, selon une première longueur L3 supérieure d'au moins 8 mm environ, de préférence supérieure d'au moins 15 mm environ, à la longueur totale L6 du manchon de guidage 4.

En pratique, lors de l'utilisation du dispositif selon le premier mode de réalisation de l'invention, le praticien utilise un unique manchon de guidage 4 qu'il dispose à coulissement sur le tronçon intermédiaire 5c d'un foret 5. Le manchon de guidage 4 est disposé sur le foret 5 avec son extrémité distale 4b en butée contre le tronçon distal de coupe 50b.

Le foret 5 est ensuite monté sur la pièce à main dentaire 6 munie de moyens de butée 13.

Les moyens de butée 13 sont réglables en hauteur, en l'espèce à l'aide d'une crémaillère 13a. On peut également utiliser des moyens de butée 13 à hauteur fixe tels que ceux décrits dans la demande de brevet français FR 2 880 534 A1 par exemple.

Après avoir réglé la hauteur des moyens de butée 13, le praticien amène le foret 5 en position sensiblement coaxiale avec le canon tubulaire de perçage 2 du guide chirurgical 1 (figure 3). Le praticien déplace ensuite le foret 5 selon la direction axiale I-I selon le mouvement illustré par la flèche 7.

Le tronçon distal de coupe 50b du foret 5 pénètre dans le canon tubulaire de perçage 2. Puis le manchon de guidage 4 s'engage à faible jeu dans le canon tubulaire de perçage 2, et assure un centrage et un guidage du foret 5 avant même que celui-ci ne touche la mâchoire 30 du patient.

En cas de léger défaut de coaxialité du manchon de guidage 4 par rapport au canon tubulaire de perçage 2 avant l'engagement, les moyens de pénétration 8 et les moyens de frottement 9 facilitent l'engagement du manchon de guidage 4 dans le canon tubulaire de perçage 2 et évitent que celui-ci ne remonte ) trop facilement le long du foret 5.

Une fois coaxial au canon tubulaire de perçage 2, le manchon de guidage 4 poursuit sa pénétration dans le canon tubulaire de perçage 2 au fur et à mesure de la progression du foret 5 dans la mâchoire 30 du patient. L'extrémité distale 4b du manchon de guidage 4 est toujours au voisinage immédiat ou à proximité du tronçon distal de coupe 50b.

Après une certaine pénétration du foret 5 dans la mâchoire 30 du patient, le manchon de guidage 4 vient en butée selon son épaulement 12 contre l'extrémité supérieure d'ouverture 2b du canon tubulaire de perçage 2.

Le manchon de guidage 4 étant monté à coulissement sur le tronçon intermédiaire 5c du foret 5, le foret 5 poursuit son mouvement axial illustré par la flèche 7, et ce jusqu'à ce que les moyens de butée 13 prennent appui sur l'extrémité proximale 4a du manchon de guidage 4 ou sur l'extrémité supérieure d'ouverture 2b du canon tubulaire de perçage 2. Le foret 5 ne peut alors plus poursuivre son mouvement axial illustré par la flèche 7, et la profondeur de forage souhaitée est atteinte.

Le praticien peut alors retirer axialement le foret 5 selon un mouvement opposé à celui illustré par la flèche 7 afin de régler à nouveau les moyens de butée 13 pour autoriser le foret 5 à creuser plus profondément.

Une fois que la profondeur de perçage satisfaisante a été atteinte à l'aide du foret 5 (en une ou plusieurs passes), le praticien peut changer de foret 5 en utilisant un autre foret 5 à tronçon distal de coupe 50b de plus grand diamètre extérieur d7 afin d'agrandir progressivement le trou dans la mâchoire du patient.

Pour ce faire, le praticien retire le manchon de guidage 4 du foret 5 de faible diamètre pour l'installer sur le tronçon intermédiaire 5c d'un autre foret 5 de diamètre d7 supérieur.

### Exemple 1 : forage d'un trou dans la mâchoire du patient avec un dispositif du premier mode de réalisation

Dans cet exemple, on choisit :
- h1 égale à 9 mm,
- L1 égale à 5 mm,
- L2 égale à 8 mm,
- L3 égale à 30 mm,
- L6 égale à 10 mm environ,
- d1 égal à 4,2 mm,
- d2 égal à 4,17 mm,
- d3 égal à 2,8 mm;
- d4 égal à 2,8 mm (+ jeu),
- d5 égal à 2,8 mm (+ jeu),
- d6 égal à 4,6 mm.

Le praticien monte le manchon de guidage 4 sur le tronçon intermédiaire 5c avec l'extrémité distale 4b en butée contre le tronçon distal de coupe 50b, puis fixe le foret 5 sur la pièce à main dentaire 6 munie de moyens de butée 13. Le praticien règle ensuite la hauteur des moyens de butée 13 pour forer un trou de profondeur déterminée.

Lorsque le foret 5 vient au contact de l'os maxillaire du patient, le manchon de guidage 4 est déjà engagé de 4 mm environ dans le canon tubulaire de perçage 2. On assure ainsi un guidage longitudinal du foret 5 avant même le début du forage, le manchon de guidage 4 étant engagé dans le canon tubulaire de perçage 2 selon une distance à peu près sensiblement égale ou supérieure à son diamètre extérieur d2.

Lorsque le foret 5 a pénétré de 4 mm environ dans l'os maxillaire, l'épaulement 12 est en butée contre l'extrémité d'ouverture supérieure 2b : le manchon de guidage 4 est immobilisé par rapport au canon de perçage tubulaire 2 avec son extrémité distale 4b située 1 mm au-dessus de la zone d'opération.

Le praticien poursuit alors le forage du trou en faisant coulisser le tronçon intermédiaire 5c du foret 5 dans le manchon de guidage 4 jusqu'à atteindre la profondeur finale du trou lorsque les moyens de butée 13 viennent en butée contre l'extrémité proximale 4a ou contre l'extrémité supérieure d'ouverture 2b du canon tubulaire de perçage 2 : le foret 5 ne peut alors plus s'enfoncer plus loin dans l'os maxillaire.

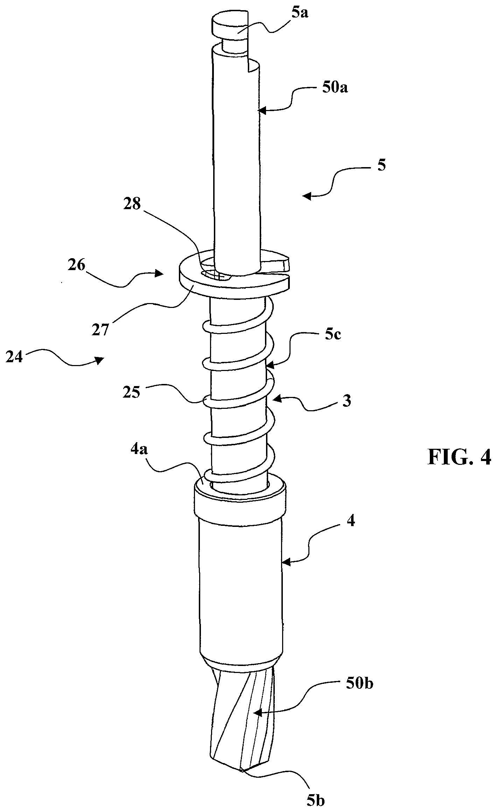

En complément ou à la place des moyens de frottement 9, on peut prévoir, comme illustré sur la figure 4, des moyens de rappel élastique 24 sollicitant en permanence le manchon de guidage 4 en coulissement vers l'extrémité distale 5b libre du foret 5.

Les moyens de rappel élastique 24 comportent un ressort hélicoïdal 25 engagé sur le tronçon intermédiaire 5c. Le ressort hélicoïdal 25 est en appui contre une butée supérieure 26 et l'extrémité proximale 4a du manchon de guidage 4. La butée supérieure 26 est réalisée de façon simple au moyen d'un circlip extérieur 27 engagé dans une gorge annulaire 28. Une telle butée supérieure 26 est facile et rapide à démonter et remonter par le praticien.

Les moyens de rappel élastique 24 facilitent et sécurisent l'engagement du manchon de guidage 4 dans le canon tubulaire de perçage 2, pour garantir que le foret 5 reste coaxial au canon tubulaire de perçage 2 lors du retrait du foret 5 hors de la mâchoire, pour ne pas détériorer les parois du trou réalisé.

Considérons désormais les figures 5 à 9 qui illustrent un dispositif de centrage et de guidage selon un second mode de réalisation de l'invention.

Dans ce second mode de réalisation, le manchon de guidage 4 est déplaçable par rapport aux moyens de support 3 entre une position basse (figure 5) et une position haute (figure 6).

Les moyens de support 3 sont réglables, rapportés et fixés sur la pièce à main dentaire 6 (figure 8) et permettent de régler la position axiale du manchon de guidage 4 par rapport au foret 5 lorsque le manchon de guidage 4 est en position basse. En position haute, des premiers moyens de butée 14 empêchent le manchon de guidage 4 de se déplacer plus encore au-delà de la position haute dans les sens et direction définis par la flèche 23 (figure 6).

Sur les figures 5 et 6, on voit plus particulièrement que des moyens élastiques 15, portés par les moyens de support 3, autorisent le déplacement axial du manchon de guidage 4 entre sa position basse (figure 5) et sa position haute (figure 6). Les moyens élastiques 15 rappellent en permanence le manchon de guidage 4 vers la position basse en direction de l'extrémité distale 5b libre du foret 5.

Comme représenté sur la figure 7, le manchon de guidage 4 comporte des moyens de pénétration 8 pour faciliter son engagement dans le canon tubulaire de perçage 2. En l'espèce, le manchon de guidage 4 comporte une extrémité distale 4b biseautée avec un chanfrein d'angle α qui peut avantageusement être choisi de 30° environ.

Tout comme dans le premier mode de réalisation de l'invention, le guide chirurgical 1 maintient l'extrémité supérieure d'ouverture 2b du canon tubulaire de perçage 2 à une première hauteur h1 déterminée de l'extrémité distale 29a de l'implant 29 en fin de vissage dans la mâchoire 30 du patient, la première hauteur h1 étant de préférence de 9 mm environ (figure 10). Pour éviter que le manchon de guidage 4 vienne en conflit avec la zone opérée de la mâchoire du patient, et pour garantir une meilleure hygiène, le manchon de guidage 4 a une longueur axiale L5 inférieure à la première hauteur h1 (figure 7). La longueur axiale L5 est avantageusement comprise entre 4 mm environ et 8 mm environ.

On voit plus particulièrement sur la figure 7 que le manchon de guidage 4 est solidaire, à son extrémité proximale 4a, de seconds moyens de butée 16 destinés à venir en appui contre l'extrémité supérieure d'ouverture 2b du canon tubulaire de perçage 2 en fin de pénétration du manchon de guidage 4 dans le canon tubulaire de perçage 2. En l'espèce, les seconds moyens de butée 16 comprennent une plaque de butée 16a s'étendant radialement à l'écart de l'axe longitudinal III-III du manchon de guidage tubulaire 4.

Sur les figures 5 et 6, les premiers moyens de butée 14 comprennent une plaque de support 17, parallèle à la plaque de butée 16a et située dans un plan décalé longitudinalement par rapport à la plaque de butée 16a.

Les moyens de support 3 comprennent deux colonnes de guidage 18a et 18b parallèles, qui assurent une liaison glissière entre la plaque de support 17 et la plaque de butée 16a.

Les moyens élastiques 15 comprennent deux ressorts hélicoïdaux 19a et 19b, intercalés entre la plaque de butée 16a et la plaque de support 17, et rappelant la plaque de support 17 et la plaque de butée 16a l'une à l'écart de l'autre. Les moyens de support 3 comprennent une tige de support 19 longitudinale, solidaire de la plaque de support 17 et destinée à être fixée sur la pièce à main dentaire 6.

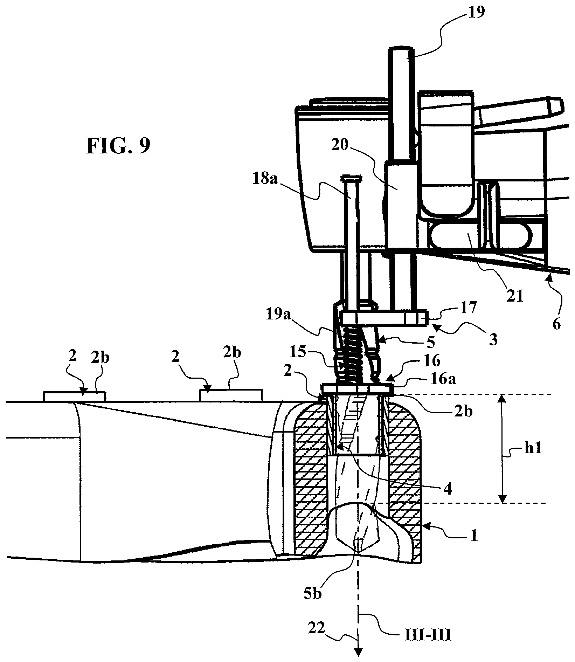

La tige de support 19 longitudinale est une tige à crémaillère, et la pièce à main dentaire 6 comporte un manchon de réception 20 longitudinal de la tige de support 19 longitudinale (figure 9). La pièce à main dentaire 6 comporte en outre des moyens de blocage 21 coopérant avec la crémaillère de la tige de support 19 longitudinale.

Afin de recevoir à faible jeu la tige de support 19 longitudinale, le manchon de réception 20 longitudinal comporte un alésage interne à section transversale complémentaire en forme de la section transversale de la tige de support 19 longitudinale.

Les sections transversales de la tige de support 19 longitudinal et de l'alésage interne du manchon de réception 20 longitudinal empêchent tout mouvement de rotation relatif entre ceux-ci.

En l'espèce, on voit sur la figure 5 que la tige de support 19 longitudinale est pourvue, sur toute sa longueur, d'un méplat 19c.

L'utilisation du dispositif de centrage et de guidage selon le second mode de réalisation de l'invention sera mieux comprise à l'aide de la description suivante.

) Lorsqu'il souhaite effectuer un trou dans la mâchoire d'un patient, le praticien monte le dispositif de centrage et de guidage représenté sur les figures 5 et 6 sur la pièce à main dentaire 6 en engageant la tige de support 19 longitudinale dans le manchon de réception 20 (figure 9). Le praticien monte alors le premier foret 5 qu'il veut utiliser pour commencer la réalisation d'un trou.

Une fois le foret 5 monté, le praticien effectue le réglage des moyens de support 3 au moyen de la crémaillère de la tige de support 19 longitudinale et des moyens de blocage 21 pour régler la position axiale du manchon de guidage 4 par rapport au foret lorsque le manchon de guidage 4 est en position basse (figure 5).

Les moyens élastiques 15 autorisent un déplacement axial connu et déterminé du manchon de guidage 4 entre sa position basse et sa position haute. Ainsi, en réglant avec précision la position basse du manchon de guidage 4, en connaissant la valeur du déplacement axial du manchon de guidage 4 entre sa position basse et sa position haute et la première hauteur h1, on connaît avec précision la profondeur de forage que peut effectuer le foret 5.

De façon pratique, on choisit des moyens élastiques 15 et des moyens de support 3 tels que le manchon de guidage 4 est déplaçable sur les moyens de support 3 selon une course axiale d'au moins 15 mm environ entre la position basse et la position haute. On peut ainsi utiliser le dispositif de centrage et de guidage selon l'invention pour effectuer l'installation d'implants entrant jusqu'à 15 mm au moins dans l'os de la mâchoire du patient, ce qui est le standard le plus grand présent sur le marché.

Une fois la position axiale du manchon de guidage 4 réglée, le praticien engage le foret 5 et le manchon de guidage 4 dans le canon tubulaire de perçage 2 selon la direction axiale III-III (figure 8) par un mouvement de sens et de direction définis par la flèche 22.

Au cours de ce mouvement, la plaque de butée 16a vient en appui contre l'extrémité supérieure d'ouverture 2b du canon tubulaire de perçage 2 (figure 9). Le praticien poursuit alors le forage de la mâchoire du patient en comprimant les moyens élastiques 15 et en effectuant la totalité du déplacement axial d'au moins 15 mm autorisé par ceux-ci. En fin de déplacement axial, le manchon de guidage 4 est en position haute et la profondeur du trou est atteinte : le praticien peut alors retirer le foret 5 et le manchon de guidage 4 du canon tubulaire de perçage 2 pour poursuivre le forage avec un autre foret 5 de diamètre supérieur.

### Exemple 2 : Réglage du dispositif selon le second mode de réalisation pour le forage d'un trou de 8 mm de profondeur dans la mâchoire du patient

Dans cet exemple, la première hauteur h1 est de 9 mm, le déplacement axial autorisé par les moyens élastiques 15 est de 15 mm, et la longueur axiale L5 du manchon de guidage 4 est de 8 mm.

Afin de régler convenablement la position axiale du manchon de guidage 4, le praticien monte le foret 5 et le dispositif de centrage et de guidage selon le second mode de réalisation de l'invention sur la pièce à main dentaire 6. La crémaillère de la tige de support 19 est bloquée dans le manchon de réception 20 de telle sorte que, lorsque les moyens élastiques 15 sont comprimés (manchon de guidage 4 en position haute), l'extrémité proximale 4a du manchon de guidage est distante de l'extrémité distale 5b du foret 5 d'une distance égale à la somme de la première hauteur h1 et de la profondeur du trou souhaitée (8 mm dans cet exemple), soit 17 mm en l'espèce.

En position basse, l'extrémité proximale 4a du manchon de guidage 4 se trouve ainsi décalée de l'extrémité distale 5b du foret 5 de 2 mm en direction de l'extrémité proximale 5a du foret 5. En position basse, l'extrémité distale 5b du foret 5 est donc contenue dans le manchon de guidage 4.

### Exemple 3 : Réglage du dispositif selon le second mode de réalisation pour le forage d'un trou de 15 mm de profondeur dans la mâchoire du patient

Dans cet exemple, la première hauteur h1 est de 9 mm, le déplacement axial autorisé par les moyens élastiques 15 est de 15 mm, et la longueur axiale L5 du manchon de guidage 4 est de 8 mm.

Afin de régler convenablement la position axiale du manchon de guidage 4, le praticien monte le foret 5 et le dispositif de centrage et de guidage selon le second mode de réalisation de l'invention sur la pièce à main dentaire 6. La crémaillère de la tige de support 19 est bloquée dans le manchon de réception 20 de telle sorte que, lorsque les moyens élastiques 15 sont comprimés (manchon de guidage 4 en position haute), l'extrémité proximale 4a du manchon de guidage est distante de l'extrémité distale 5b du foret 5 d'une distance égale à la somme de la première hauteur h1 et de la profondeur du trou souhaitée (15 mm dans cet exemple), soit 24 mm en l'espèce.

En position basse, l'extrémité proximale 4a du manchon de guidage 4 se trouve ainsi décalée de l'extrémité distale 5b du foret 5 de 9 mm en direction de l'extrémité proximale 5a du foret 5. En position basse, l'extrémité distale 5b du foret 5 dépasse ainsi de 1 mm au-delà de l'extrémité distale 4b du manchon de guidage 4.

On constate que, dans chacun des exemples 2 et 3 expliqués ci-dessus, le manchon de guidage 4, dont la longueur est de 8 mm, est toujours engagé d'au moins 4 mm dans le canon tubulaire de perçage 2 avant même que le foret 5 ne commence à creuser l'os de la mâchoire du patient. On garantit ainsi un guidage efficace du foret 5 avant même le début du forage, le manchon de guidage 4 étant engagé dans le canon tubulaire de perçage 2 selon une distance à peu près sensiblement égale ou supérieure à son diamètre extérieur d2 (qui est de 4,17 mm ou 5,17 mm selon les standards les plus courants disponibles sur le marché). Ceci reste également le cas pour des réglages identiques et un manchon de guidage 4 de longueur L5 de 4 mm.

La présente invention n'est pas limitée aux modes de réalisation qui ont été explicitement décrits, mais elle en inclut les diverses variantes et généralisations contenues dans l'étendue de protection définie par les revendications ci-après.

## Revendications

1. Dispositif de centrage et de guidage d'au moins un foret (5) de pièce à main dentaire (6) lors du forage d'un trou pour un implant (29) dans la mâchoire (30) d'un patient en coopération avec un guide chirurgical (1) muni d'un canon tubulaire de perçage (2) à extrémité supérieure d'ouverture (2b), comportant :
- un manchon de guidage (4) tubulaire, apte à s'engager à faible jeu dans le canon tubulaire de perçage (2),
- au moins un foret (5) comportant une extrémité proximale (5a) et une extrémité distale libre (5b), avec un tronçon proximal de connexion (50a) à la pièce à main dentaire (6) et un tronçon distal de coupe (50b),
- des moyens de support (3) maintenant en permanence le manchon de guidage (4) tubulaire en position coaxiale et à coulissement par rapport audit au moins un foret (5) pour favoriser l'engagement du manchon de guidage (4) dans le canon tubulaire de perçage (2) au plus tard dès que ledit au moins un foret (5) commence à forer la mâchoire (30) du patient, lesdits moyens de support (3) comprenant, sur ledit au moins un foret (5), un tronçon intermédiaire (5c) cylindrique lisse de diamètre extérieur (d3) sensiblement égal ou peu inférieur au diamètre intérieur (d4) du manchon de guidage (4) et sur lequel est engagé à coulissement le manchon de guidage (4) tubulaire,
**caractérisé en ce que** le manchon de guidage (4) comporte des moyens de frottement (9) sur le tronçon intermédiaire (5c) cylindrique lisse lors de son coulissement sur celui-ci.

2. Dispositif selon la revendication 1, **caractérisé en ce que** les moyens de frottement (9) comportent un joint torique (10) de diamètre intérieur (d5) peu inférieur ou sensiblement égal au diamètre extérieur (d3) du tronçon intermédiaire (5c) cylindrique lisse et logé dans une gorge annulaire intérieure (11) du manchon de guidage (4).

3. Dispositif selon l'une des revendications 1 ou 2, **caractérisé en ce qu'**il comprend une pluralité de forets (5) à tronçon distal de coupe (50b) de différents diamètres (d7) et comprenant tous un tronçon intermédiaire (5c) cylindrique lisse de même diamètre extérieur (d3) sensiblement égal ou peu inférieur au diamètre intérieur (d4) du manchon de guidage (4).

4. Dispositif selon l'une quelconque des revendications 1 à 3, **caractérisé en ce que** le tronçon intermédiaire (5c) cylindrique lisse s'étend, depuis le tronçon distal de coupe (50b), selon une première longueur (L3) supérieure d'au moins 8 mm environ, de préférence supérieure d'au moins 15 mm environ, à la longueur totale (L6) du manchon de guidage (4).

5. Dispositif selon l'une quelconque des revendications 1 à 4, **caractérisé en ce que** le manchon de guidage (4) comporte des moyens de pénétration (8) qui facilitent son engagement dans le canon tubulaire de perçage (2), de préférence une extrémité distale (4b) biseautée.

6. Dispositif selon l'une quelconque des revendications 1 à 5, **caractérisé en ce que** :
- il comporte un guide chirurgical (1) muni d'un canon tubulaire de perçage (2) à extrémité supérieure d'ouverture (2b),
- en position sur la mâchoire (30) du patient, le guide chirurgical (1) maintient l'extrémité supérieure d'ouverture (2b) du canon tubulaire de perçage (2) à une première hauteur (h1) déterminée de l'extrémité distale (29a) de l'implant (29) en fin de vissage dans la mâchoire (30) du patient, la première hauteur (h1) étant de préférence de 9 mm environ,
- le tronçon distal de coupe (50b) a une longueur axiale (L1) inférieure à la première hauteur (h1), comprise de préférence entre environ 4 mm et 7 mm environ,
- le tronçon distal de coupe (50b) a un diamètre extérieur (d7) supérieur au diamètre extérieur (d3) du tronçon intermédiaire lisse (5c).

7. Dispositif selon l'une quelconque des revendications 1 à 6, **caractérisé en ce que** :
- il comporte un guide chirurgical (1) muni d'un canon tubulaire de perçage (2) à extrémité supérieure d'ouverture (2b),
- en position sur la mâchoire (30) du patient, le guide chirurgical (1) maintient l'extrémité supérieure d'ouverture (2b) du canon tubulaire de perçage (2) à une première hauteur (h1) déterminée de l'extrémité distale (29a) de l'implant (29) en fin de vissage dans la mâchoire (30) du patient, la première hauteur (h1) étant de préférence de 9 mm environ,
- le manchon de guidage (4) comporte une extrémité proximale (4a) de diamètre extérieur (d6) supérieur au diamètre intérieur (d1) du canon tubulaire de perçage (2), avec un épaulement (12) à face de butée (12a) orientée en direction de son extrémité distale (4b) et destiné à venir en appui contre l'extrémité supérieure d'ouverture (2b) du canon tubulaire de perçage (2) en fin de pénétration du manchon de guidage (4) dans le canon tubulaire de perçage (2),
- la face de butée (12a) de l'épaulement (12) du manchon de guidage (4) est distante de l'extrémité distale (4b) du manchon de guidage (4) d'une longueur (L2) inférieure à la première hauteur (h1).

8. Dispositif selon la revendication 7, **caractérisé en ce que** la face de butée (12a) de l'épaulement (12) du manchon de guidage (4) est distante de l'extrémité distale (4b) du manchon de guidage (4) d'une longueur (L2) comprise entre environ 4 mm et environ 8 mm, et de préférence de 8 mm.

9. Dispositif selon l'une quelconque des revendications 1 à 8, **caractérisé en ce qu'**il comporte en outre des moyens réglables de limitation de la profondeur de pénétration du foret (5) dans la mâchoire (30) du patient, de préférence des moyens de butée (13) rapportés et fixés sur la pièce à main dentaire (6) et destinés à venir en appui sur l'extrémité proximale (4a) du manchon de guidage (4) ou sur l'extrémité supérieure d'ouverture (2b) du canon tubulaire de perçage (2) en fin de pénétration du foret (5) dans la mâchoire (30) du patient.

10. Dispositif selon l'une quelconque des revendications 1 à 9, **caractérisé en ce qu'**il comprend en outre des moyens de rappel élastique (24) sollicitant en permanence le manchon de guidage 4 en coulissement vers l'extrémité distale (5b) libre du foret (5).

## Patentansprüche

1. Vorrichtung zum Zentrieren und Führen wenigstens eines Bohrers (5) für ein Handstück (6) beim Bohren einer Kavität für ein Implantat (29) im Kiefer (30) eines Patienten zusammen mit einer chirurgischen Führung (1), die mit einer röhrenförmigen Bohrbuchse (2) mit einem geöffneten oberen Ende (2b) ausgestattet ist, umfassend:
- ein röhrenförmiger Führungsstutzen (4), der eingerichtet ist, mit einem leichten Spiel in die röhrenförmige Bohrbuchse (2) einzugreifen,
- wenigstens ein Bohrer (5), der ein proximales Ende (5a) und ein distales Ende (5b) mit einem proximalen Verbindungsabschnitt (50a) am Handstück (6) und einem distalen Schneidabschnitt (50b) umfasst,
- Tragemittel (3), die den rohrförmigen Führungsstutzen (4) dauerhaft in einer relativ zum wenigstens einen Bohrer (5) koaxialen und verschieblichen Position halten, um den Eingriff des rohrförmigen Führungsstutzens (4) in die rohrförmige Bohrbuchse (2) zu unterstützen, sobald der genannte wenigstens eine Bohrer (5) beginnt, in den Kiefer (30) des Patienten zu bohren, wobei die genannten Tragemittel (3) über dem wenigstens einen Bohrer (5) einen glatten zylindrischen Zwischenabschnitt (5c) mit einem Außendurchmesser (d3) umfassen, der im Wesentlichen gleich oder geringfügig kleiner ist, als ein Innendurchmesser (d4) des rohrförmigen Führungsstutzens (4) und über den der rohrförmige Führungsstutzen (4) gleitend in Eingriff steht, **dadurch gekennzeichnet, dass** der rohrförmige Führungsstutzen (4) Reibmittel (9) umfasst, die eingerichtet sind, auf dem glatten zylindrischen Zwischenabschnitt (5c) zu gleiten.

2. Vorrichtung nach Anspruch 1, **dadurch gekennzeichnet, dass** die Reibmittel (9) einen O-Ring (10) mit einem Innendurchmesser (d5) umfassen, der geringfügig kleiner oder im Wesentlichen gleich dem Außendurchmesser (d3) des glatten zylindrischen Zwischenabschnitts (5c) ist und der in einer ringförmigen nach innen gerichteten Rille (11) des Führungsstutzens (4) angeordnet ist.

3. Vorrichtung nach einem der Ansprüche 1 oder 2, **dadurch gekennzeichnet, dass** sie eine Vielzahl an Bohrern (5) mit distalen Schneidabschnitten (50b) unterschiedlicher Durchmesser (d7) und alle enthaltend einen glatten zylindrischen Zwischenabschnitt (5c) einen Außendurchmesser (d3) besitzen, der im Wesentlichen gleich oder geringfügig kleiner ist, als einen Innendurchmesser (d4) des Führungsstutzens (4).

4. Vorrichtung nach einem der Ansprüche 1 bis 3, **dadurch gekennzeichnet, dass** sich der glatte zylindrische Zwischenabschnitt (5c) vom distalen Schneidabschnitt (50b) über eine erste Länge (L3) erstreckt, die größer ist, als die Gesamtlänge (L6) des Führungsstutzens (4) mit mindestens als ungefähr 8mm, vorzugsweise mit mindestens mehr als ungefähr 15mm.

5. Vorrichtung nach einem der Ansprüche 1 bis 4, **dadurch gekennzeichnet, dass** der Führungsstutzen (4) zur Unterstützung seines Eingriffs in die röhrenförmige Bohrbuchse (2) Durchdringungsmittel (8) vorzugsweise mit einem abgeschrägten distalen Ende (4b) umfasst.

6. Vorrichtung nach einem der Ansprüche 1 bis 5, **dadurch gekennzeichnet, dass**:
- sie eine chirurgische Führung (1) umfasst, die mit einer röhrenförmigen Bohrbuchse (2) mit einem geöffneten oberen Ende (2b) ausgestattet ist,
- die chirurgische Führung (1) in Position über dem Kiefer (30) des Patienten das geöffnete obere Ende (2b) der röhrenförmigen Bohrbuchse (2) auf einer vorbestimmten ersten Höhe (h1) über dem distalen Ende (29a) des Implantats (29) endseitig eines Schraubgewindes durch den Kiefer (30) des Patienten hält, wobei die erste Höhe (h1) vorzugsweise ungefähr 9mm beträgt,
- der distale Schneidabschnitt (50b) eine axiale Länge (L1) besitzt, die kleiner ist, als die erste Höhe (h1), vorzugsweise zwischen ungefähr 4mm und ungefähr 7mm,
- der distale Schneidabschnitt (50b) einen Außendurchmesser (d7) besitzt, der größer ist, als ein Außendurchmesser (d3) des zylindrischen Zwischenabschnittes (5c).

7. Vorrichtung nach einem der vorstehenden Ansprüche 1 bis 6, **dadurch gekennzeichnet, dass**:
- sie eine chirurgische Führung (1) umfasst, die mit einer röhrenförmigen Bohrbuchse (2) mit einem geöffneten oberen Ende (2b) ausgestattet ist,
- die chirurgische Führung (1) in Position über dem Kiefer (30) des Patienten das geöffnete obere Ende (2b) der röhrenförmigen Bohrbuchse (2) auf einer vorbestimmten ersten Höhe (h1) über dem distalen Ende (29a) des Implantats (29) endseitig eines Schraubgewindes durch den Kiefer (30) des Patienten hält, wobei die erste Höhe (h1) vorzugsweise ungefähr 9mm beträgt,
- der Führungsstutzen (4) ein proximales Ende (4a) umfasst, mit einem Außendurchmesser (d6), der größer ist als ein Innendurchmesser (d1) der röhrenförmigen Bohrbuchse (2) und mit einer Schulter (12) mit einer Aufsetzfläche (12a), die in die Richtung seines distalen Endes (4b) gerichtet und vorgesehen ist, gegen das offene obere Ende (2b) der rohrförmigen Bohrbuchse (2) gestützt zu werden, wenn der Führungsstutzen (4) die Bohrbuchse (2) durchdrungen hat,
- die Aufsetzfläche (12a) der Schulter (12) des Führungsstutzens (4) vom distalen Ende (4b) des Führungsstutzens (4) einen Abstand (L2) von weniger als die erste Höhe (h1) besitzt.

8. Vorrichtung nach Anspruch 7, **dadurch gekennzeichnet, dass** der Abstand (L2) der Aufsetzfläche (12a) der Schulter (12) des Führungsstutzens (4) vom distalen Ende (4b) des Führungsstutzens (4) zwischen ungefähr 4mm und ungefähr 8mm und vorzugsweise 8mm beträgt.

9. Vorrichtung nach einem der vorstehenden Ansprüche 1 bis 8, **dadurch gekennzeichnet, dass** sie unter anderem einstellbare Mittel zur Beschränkung der Tiefe des Eindringens des Bohrer (5) in den Kiefer (30) des Patienten und Anschlagmittel (13) umfasst, die am dentalen Handstück (6) befestigt und eingerichtet sind, auf das proximale Ende (4a) des Führungsstutzens (4) oder auf das obere Ende (2b) der röhrenförmigen Bohrbuchse (2) am Ende des Endringens des Bohrer (5) in den Kiefer (30) des Patienten aufgesetzt zu werden.

10. Vorrichtung nach einem der vorstehenden Ansprüche 1 bis 9, **dadurch gekennzeichnet, dass** sie unter anderem elastische Rückstellmittel (24) umfasst, die stetig den gleitenden Führungsstutzen 4 gegen das freie distale Ende (5b) des Bohrer (5) drücken.

## Claims

1. Device for centering and guiding at least one drill bit (5) fitted to a dental handpiece (6) while drilling a hole for an implant (29) in the jaw (30) of a patient in cooperation with a surgical guide (1) provided with a tubular drilling bush (2) with an open upper end (2b), comprising :
- a tubular guide sleeve (4), adapted to enter with a small clearance in the tubular drilling bush (2),
- at least a drill bit (5) having a proximal end (5a) and a distal free end (5b), with a proximal section (50a) for connecting it to the dental handpiece (6) and a distal cutting section (50b),
- support means (3) holding the tubular guide sleeve (4) permanently coaxial with and slidable relative to said at least one drill bit (5) to enable the engagement of the guide sleeve (4) in the tubular drilling bush (2) at the latest as soon as said at least one drill bit (5) begins to drill the jaw (30) of the patient, said support means (3) comprising, on the said at least one drill bit (5), a smooth cylindrical intermediate section (5c) with an outside diameter (d3) substantially equal to or slightly less than the inside diameter (d4) of the guide sleeve (4) and on which the tubular guide sleeve (4) is slidably engaged,
**characterized in that** the guide sleeve (4) includes friction means (9) adapted to slide on the smooth cylindrical intermediate section (5c).

2. Device according to claim 1, **characterized in that** the friction means (9) include an O-ring (10) having an inside diameter (d5) slightly less than or substantially equal to the outside diameter (d3) of the smooth cylindrical intermediate section (5c) and housed in an interior annular groove (11) of the guide sleeve (4).

3. Device according to either of claims 1 or 2, **characterized in that** it includes a plurality of drill bits (5) with distal cutting sections (50b) of different diameters (d7) and all including a smooth cylindrical intermediate section (5c) of the same outside diameter (d3) substantially equal to or slightly less than the inside diameter (d4) of the guide sleeve (4).

4. Device according to any of claims 1 to 3, **characterized in that** the smooth cylindrical intermediate section (5c) extends over a first length (L3) from the distal cutting section (50b) greater than the total length (L6) of the guide sleeve (4) by at least approximately 8 mm, preferably at least approximately 15 mm.

5. Device according to any of claims 1 to 4, **characterized in that** the guide sleeve (4) includes penetration means (8) to facilitate its engagement in the tubular drilling bush (2), preferably in the form of a bevelled distal end (4b).

6. Device according to any of claims 1 to 5, **characterized in that** :
- it comprises a surgical guide (1) provided with a tubular drilling bush (2) with an open upper end (2b),
- when in position on the jaw (30) of the patient, the surgical guide (1) holds the open upper end (2b) of the tubular drilling bush (2) at a particular first height (h1) above the distal end (29a) of the implant (29) at the end of screwing it into the jaw (30) of the patient, the first height (h1) being preferably approximately 9 mm,
- the distal cutting section (50b) has an axial length (L1) less than the first height (h1), preferably between approximately 4 mm and approximately 7 mm inclusive,
- the distal cutting section (50b) has an outside diameter (d7) greater than the outside diameter (d3) of the smooth intermediate section (5c).

7. Device according to any of claims 1 to 6, **characterized in that** :
- it comprises a surgical guide (1) provided with a tubular drilling bush (2) with an open upper end (2b),
- when in position on the jaw (30) of the patient, the surgical guide (1) holds the open upper end (2b) of the tubular drilling bush (2) at a particular first height (h1) above the distal end (29a) of the implant (29) at the end of screwing it into the jaw (30) of the patient, the first height (h1) preferably being approximately 9 mm,
- the guide sleeve (4) has a proximal end (4a) with an outside diameter (d6) greater than the inside diameter (d1) of the tubular drilling bush (2) and with a shoulder (12) with an abutment face (12a) oriented in the direction of its distal end (4b) and intended to come to bear against the open upper end (2b) of the tubular drilling bush (2) at the end of penetration of the guide sleeve (4) into the tubular drilling bush (2),
- the abutment face (12a) of the shoulder (12) of the guide sleeve (4) is at a distance (L2) less than the first height (h1) from the distal end (4b) of the guide sleeve (4).

8. Device according to claim 7, **characterized in that** the abutment face (12a) of the shoulder (12) of the guide sleeve (4) is at a distance (L2) between approximately 4 mm and approximately 8 mm, and preferably 8 mm, from the distal end (4b) of the guide sleeve (4).

9. Device according to any of claims 1 to 8, **characterized in that** it further includes adjustable means for limiting the depth of penetration of the drill bit (5) in the jaw (30) of the patient, preferably abutment means (13) fastened to the dental handpiece (6) and adapted to come to bear on the proximal end (4a) of the guide sleeve (4) or on the open upper end (2b) of the tubular drilling bush (2) at the end of penetration of the drill bit (5) into the jaw (30) of the patient.

10. Device according to any of claims 1 to 9, **characterized in that** it further includes spring return means (24) continuously urging the sliding guide sleeve (4) toward the free distal end (5b) of the drill bit (5).
